(19) **Europäisches Patentamt / European Patent Office / Office européen des brevets**

(11) **EP 4 005 632 A1**

(12) **EUROPEAN PATENT APPLICATION**

(43) Date of publication:
01.06.2022 Bulletin 2022/22

(21) Application number: 21210152.1

(22) Date of filing: 24.11.2021

(51) International Patent Classification (IPC):
*A61N 5/10* (2006.01)  *B29C 64/386* (2017.01)
*G09B 23/30* (2006.01)  *G16H 20/40* (2018.01)
*G16H 50/50* (2018.01)

(52) Cooperative Patent Classification (CPC):
**G16H 20/40; A61N 5/1071; B29C 64/386; G09B 23/30; G16H 50/50;** A61N 5/1075; A61N 2005/1076

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 25.11.2020 KR 20200160078
12.07.2021 KR 20210091049

(71) Applicant: **Yonsei University, University-Industry Foundation(UIF).**
**Seoul 03722 (KR)**

(72) Inventors:
- **LEE, Ik Jae**
  **06273 Seoul (KR)**
- **LEE, Ho**
  **06273 Seoul (KR)**
- **CHOI, Yeon Ho**
  **06273 Seoul (KR)**
- **PARK, Kwang Woo**
  **06273 Seoul (KR)**

(74) Representative: **Pace Napoleone, Maria et al**
**De Simone & Partners S.r.l.**
**Via Vincenzo Bellini, 20**
**00198 Roma (IT)**

(54) **METHOD AND APPARATUS FOR MANUFACTURING 3D-PRINTED CHEST PHANTOM FOR INTRAOPERATIVE RADIOTHERAPY**

(57) According to the method and the apparatus for manufacturing a 3D-printed chest phantom for IORT according to the exemplary embodiment of the present disclosure, in order to perform a patient-specific quality assurance (QA) for intraoperative radiotherapy (IORT), a 3D-printed chest phantom corresponding to a patient is manufactured to validate a usefulness of the patient-specific quality assurance (PSQA) with regard to the IORT for the breast cancer.

Fig. 3

START

ACQUIRE COMPUTED TOMOGRAPHY (CT) IMAGE FOR CHEST OF TARGET PATIENT — S110

ACQUIRE INFILL RATIO FOR EVERY SUB REGION OF COMPUTED TOMOGRAPHY (CT) IMAGE BY DELINEATING CONTOUR BASED ON COMPUTED TOMOGRAPHY (CT) IMAGE — S120

PRODUCE 3D-PRINTED CHEST PHANTOM CORRESPONDING TO TARGET PATIENT BASED ON COMPUTED TOMOGRAPHY (CT) IMAGE AND INFILL RATIO FOR EVERY SUB REGION — S130

MEASURE DOSE USING SPHERICAL APPLICATOR BASED ON 3D-PRINTED CHEST PHANTOM — S140

CONFIRM QUALITY OF IORT FOR TARGET PATIENT BASED ON PREDETERMINED EXPECTED DOSE AND DOSE MEASURED FOR 3D-PRINTED CHEST PHANTOM — S150

END

EP 4 005 632 A1

Description

BACKGROUND

Field

The present disclosure relates to a method and an apparatus for manufacturing a 3D-printed chest phantom for intraoperative radiotherapy (IORT), and more particularly, to a method and an apparatus for assuring the safety and the treatment accuracy of intraoperative radiotherapy (IORT).

Description of the Related Art

According to the intraoperative radiotherapy (IORT), a single-fraction dose of highly localized radiation is directly delivered in the vicinity of the tumor during the surgery. IORT is not inferior to external-beam radiation therapy (EBRT) in terms of overall survival. An amount of tumor to be removed varies depending on the surgery result so that pretreatment planning and delivery quality assurance (DQA) are limited.
Therefore, with regard to the IORT for the breast cancer, it is necessary to verify the usefulness of patient-specific quality assurance (PSQA).

SUMMARY

An object to be achieved by the present disclosure is to provide a method and an apparatus for manufacturing a 3D-printed chest phantom for IORT to manufacture a 3D-printed chest phantom corresponding to a patient to perform a patient-specific quality assurance (QA) for the intraoperative radiotherapy (IORT).
Other and further objects of the present invention which are not specifically described can be further considered within the scope easily deduced from the following detailed description and the effect.
In order to achieve the above-described objects, according to an aspect of the present disclosure, a 3D-printed chest phantom manufacturing method for IORT includes: acquiring a computed tomography (CT) image for a chest of a target patient; acquiring an infill ratio for every sub region of the computed tomography (CT) image by delineating a contour based on the computed tomography (CT) image; producing a 3D-printed chest phantom corresponding to the target patient based on the computed tomography (CT) image and the infill ratio for every sub region.
Here, the producing of a 3D-printed chest phantom includes: acquiring a virtual object to form the 3D-printed chest phantom from the computed tomography (CT) image; acquiring a 3D object in a stereo lithography (STL) format based on the computed tomography (CT) image and the virtual object; acquiring 3D rendering data by three-dimensionally rendering a structure based on the 3D object and dividing the 3D rendering data into a plurality of sections; and producing the 3D-printed chest phantom using a 3D printing device, based on the 3D rendering data which is divided into the plurality of sections using the infill ratio for every sub region.
Here, in the dividing into the plurality of sections, the 3D rendering data is divided into the plurality of sections based on a position in which a spherical applicator is inserted, a position in which a first dose measurement film to check a dose distribution on a surface is inserted, and a position in which a second dose measurement film to check a dose distribution on a depth is inserted.
Here, a position in which the first dose measurement film is inserted is determined so as to locate the first dose measurement film on a lower horizontal plane of a spherical shape into which the spherical applicator is inserted and a position in which the second dose measurement film is inserted is determined to locate the second dose measurement film on a vertical plane below the horizontal plane.
Here, in the acquiring of an infill ratio, the infill ratio is acquired for every sub region of the computed tomography (CT) image using a predetermined conversion table representing a correlation between the infill ratio and a Hounsfield unit (HU) value.
Here, the acquiring of an infill ratio includes: acquiring a seeded region from the computed tomography (CT) image; acquiring a background region from the computed tomography (CT) image using the seeded region; acquiring an inversed seeded region using the Hounsfield unit (HU) value of the seeded region; correcting the inversed seeded region using a non-local total variation (NLTV) objective function to which a mutual information (MI) based weight is applied; adding the background region to the corrected seeded region; acquiring a plurality of sub regions by performing the K-means clustering based on the seeded region with the added background region; and acquiring a Hounsfield unit (HU) average for each of the plurality of sub regions and acquiring an infill ratio for every sub region of the computed tomography (CT) image using the conversion table.
Here, the objective function uses a weight between a voxel of the inversed seeded region and a voxel of a non-local patch included in a search set for a stationary patch and the weight includes a statistical scale value for one voxel acquired by means of the entropy and a factor which is spatially encoded for one voxel.

Here, the sub region may be one of a lung region and a soft tissue region.

In order to achieve the above-described technical objects, according to another aspect of the present disclosure, a computer program is stored in a computer readable storage medium to allow a computer to execute any one of the 3D-printed chest phantom manufacturing methods for IORT.

In order to achieve the above-described objects, according to another aspect of the present disclosure, a 3D-printed chest phantom manufacturing apparatus for IORT is a chest phantom manufacturing apparatus which manufactures a 3D-printed chest phantom for intraoperative radiotherapy (IORT), including: a memory which stores one or more programs to manufacture the 3D-printed chest phantom; and one or more processors which perform an operation for manufacturing the 3D-printed chest phantom according to the one or more programs stored in the memory, and the processor acquires a computed tomography (CT) image for a chest of a target patient, delineates a contour based on the computed tomography (CT) image to acquire an infill ratio for every sub region of the computed tomography (CT) image, and produces a 3D-printed chest phantom corresponding to the target patient based on the computed tomography (CT) image and the infill ratio for every sub region.

Here, the processor acquires a virtual object to form the 3D-printed chest phantom from the computed tomography (CT) image; acquires a 3D object in a stereo lithography (STL) format based on the computed tomography (CT) image and the virtual object, acquires 3D rendering data by three-dimensionally rendering a structure based on the 3D object and divides the 3D rendering data into a plurality of sections, and produces the 3D-printed chest phantom using a 3D printing device, based on the 3D rendering data which is divided into the plurality of sections using the infill ratio for every sub region.

Here, the processor divides the 3D rendering data into the plurality of sections based on a position in which a spherical applicator is inserted, a position in which a first dose measurement film to check a dose distribution on a surface is inserted, and a position in which a second dose measurement film to check a dose distribution on a depth is inserted.

Here, the processor acquires the infill ratio for every sub region of the computed tomography (CT) image using a predetermined conversion table representing a correlation between the infill ratio and a Hounsfield unit (HU) value.

According to the method and the apparatus for manufacturing a 3D-printed chest phantom for IORT according to the exemplary embodiment of the present disclosure, in order to perform a patient-specific quality assurance (QA) for intraoperative radiotherapy (IORT), a 3D-printed chest phantom corresponding to a patient is manufactured to verify a usefulness of the patient-specific quality assurance (PSQA) with regard to the IORT for the breast cancer.

The effects of the present invention are not limited to the technical effects mentioned above, and other effects which are not mentioned can be clearly understood by those skilled in the art from the following description

## BRIEF DESCRIPTION OF THE DRAWINGS

FIG. 1 is a block diagram for explaining a 3D-printed chest phantom manufacturing apparatus for IORT according to an exemplary embodiment of the present disclosure;

FIG. 2 is a block diagram for explaining a detailed configuration of a chest phantom manufacturing apparatus illustrated in FIG. 1;

FIG. 3 is a flowchart for explaining a 3D-printed chest phantom manufacturing method for IORT according to an exemplary embodiment of the present disclosure;

FIG. 4 is a flowchart for explaining a sub step of a step of acquiring an infill ratio illustrated in FIG. 3;

FIG. 5 is a view for explaining an example of a process of acquiring a plurality of sub regions from a computed tomography (CT) image according to an exemplary embodiment of the present disclosure;

FIG. 6 is a view for explaining an example of a process of acquiring an infill ratio for each of a plurality of sub regions illustrated in FIG. 5;

FIG. 7 is a flowchart for explaining a sub step of a step of manufacturing a 3D-printed chest phantom illustrated in FIG. 3;

FIG. 8 is a view for explaining an example of a process of manufacturing a 3D-printed chest phantom according to an exemplary embodiment of the present disclosure;

FIG. 9 is a view for explaining an example of a process of acquiring a plurality of sections illustrated in FIG. 8;

FIG. 10 is a view illustrating an example of a pseudo-code of mutual information based non-local total variation denoising according to an exemplary embodiment of the present disclosure;

FIG. 11 is a view for explaining a performance of a 3D-printed chest phantom manufacturing method for IORT according to an exemplary embodiment of the present disclosure which illustrates a result of comparing a dose measured using a patient-specific 3D-printed chest phantom and an estimated dose; and

FIG. 12 is a view for explaining a performance of a 3D-printed chest phantom manufacturing method for IORT according to an exemplary embodiment of the present disclosure which illustrates a surface dose and a depth dose which are measured using a patient-specific 3D-printed chest phantom and an estimated dose.

## DETAILED DESCRIPTION OF THE EMBODIMENT

Hereinafter, embodiments of the present disclosure will be described in detail with reference to the accompanying drawings. Advantages and characteristics of the present disclosure and a method of achieving the advantages and characteristics will be clear by referring to exemplary embodiments described below in detail together with the accompanying drawings. However, the present invention is not limited to exemplary embodiments disclosed herein but will be implemented in various different forms. The exemplary embodiments are provided by way of example only so that a person of ordinary skilled in the art can fully understand the disclosures of the present invention and the scope of the present invention. Therefore, the present invention will be defined only by the scope of the appended claims. Like reference numerals generally denote like elements throughout the specification.

Unless otherwise defined, all terms (including technical and scientific terms) used in the present specification may be used as the meaning which may be commonly understood by the person with ordinary skill in the art, to which the present invention belongs. It will be further understood that terms defined in commonly used dictionaries should not be interpreted in an idealized or excessive sense unless expressly and specifically defined. In the specification, the terms "first" or "second" are used to distinguish one component from the other component so that the scope should not be limited by these terms. For example, a first component may also be referred to as a second component and likewise, the second component may also be referred to as the first component.

In the present specification, in each step, numerical symbols (for example, a, b, and c) are used for the convenience of description, but do not explain the order of the steps so that unless the context apparently indicates a specific order, the order may be different from the order described in the specification. That is, the steps may be performed in the order as described or simultaneously, or an opposite order.

In this specification, the terms "have", "may have", "include", or "may include" represent the presence of the characteristic (for example, a numerical value, a function, an operation, or a component such as a part"), but do not exclude the presence of additional characteristic.

Hereinafter, an exemplary embodiment of a method and an apparatus for manufacturing a 3D-printed chest phantom for IORT according to the present disclosure will be described in detail with reference to the accompanying drawings.

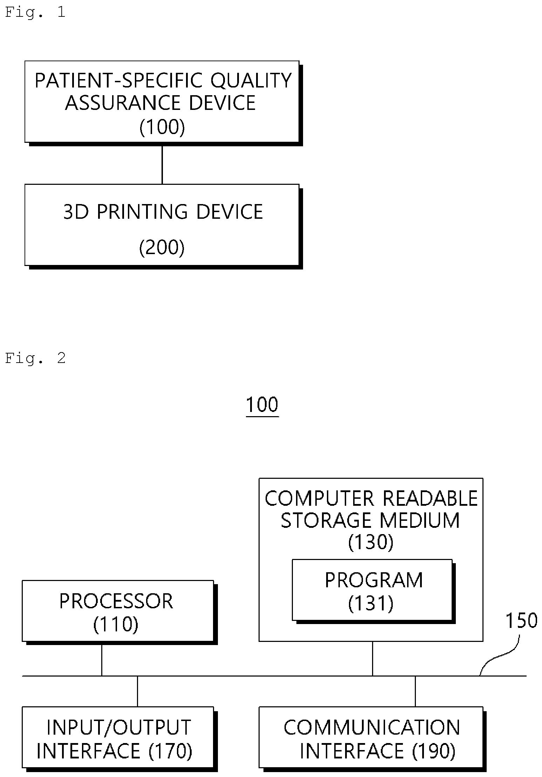

First, a 3D-printed chest phantom manufacturing apparatus for IORT according to an exemplary embodiment of the present disclosure will be described with reference to FIGS. 1 and 2.

FIG. 1 is a block diagram for explaining a 3D-printed chest phantom manufacturing apparatus for IORT according to an exemplary embodiment of the present disclosure and FIG. 2 is a block diagram for explaining a detailed configuration of a chest phantom manufacturing apparatus illustrated in FIG. 1.

Referring to FIG. 1, in order to perform patient-specific quality assurance (QA) for intraoperative radiotherapy (IORT), the 3D-printed chest phantom manufacturing apparatus 100 for IORT according to an exemplary embodiment of the present disclosure (hereinafter, simply referred to as a chest phantom manufacturing apparatus) manufactures a 3D-printed chest phantom corresponding to a patient.

For example, in order to assure the stability and the treatment accuracy of the IORT for the breast cancer of a target patient, the chest phantom manufacturing apparatus 100 may manufacture the 3D-printed chest phantom for the target patient using a 3D printing device 200. Thereafter, the quality assurance for the corresponding patient may be performed using the manufactured 3D-printed chest phantom. To this end, the chest phantom manufacturing apparatus 100 may include one or more processor 110, a computer readable storage medium 130, and a communication bus 150.

The processor 110 controls the chest phantom manufacturing apparatus 100 to operate. For example, the processor 110 may execute one or more programs 131 stored in the computer readable storage medium 130. One or more programs 131 include one or more computer executable instructions and when the computer executable instruction is executed by the processor 110, the computer executable instruction may be configured to allow the chest phantom manufacturing apparatus 100 to perform an operation for manufacturing a 3D-printed chest phantom.

The computer readable storage medium 130 is configured to store a computer executable instruction or program code, program data and/or other appropriate format of information to manufacture a 3D-printed chest phantom. The program 131 stored in the computer readable storage medium 130 includes a set of instructions executable by the processor 110. In one exemplary embodiment, the computer readable storage medium 130 may be a memory (a volatile memory such as a random access memory, a non-volatile memory, or an appropriate combination thereof), one or more magnetic disk storage devices, optical disk storage devices, flash memory devices, and another format of storage mediums which is accessed by the chest phantom manufacturing apparatus 100 and stores desired information, or an appropriate combination thereof.

The communication bus 150 includes a processor 110 and a computer readable storage medium 130 to interconnect various components of the chest phantom manufacturing apparatus 100 to each other.

The chest phantom manufacturing apparatus 100 may include one or more input/output interfaces 170 and one or more communication interfaces 190 which provide an interface for one or more input/output devices. The input/output interface 170 and the communication interface 190 are connected to the communication bus 150. The input/output device (not illustrated) may be connected to the other components of the chest phantom manufacturing apparatus 100 by means of the input/output interface 170. The 3D printing device 200 is connected to the chest phantom manufacturing apparatus 100 via the communication interface 190 to transmit and receive various data.

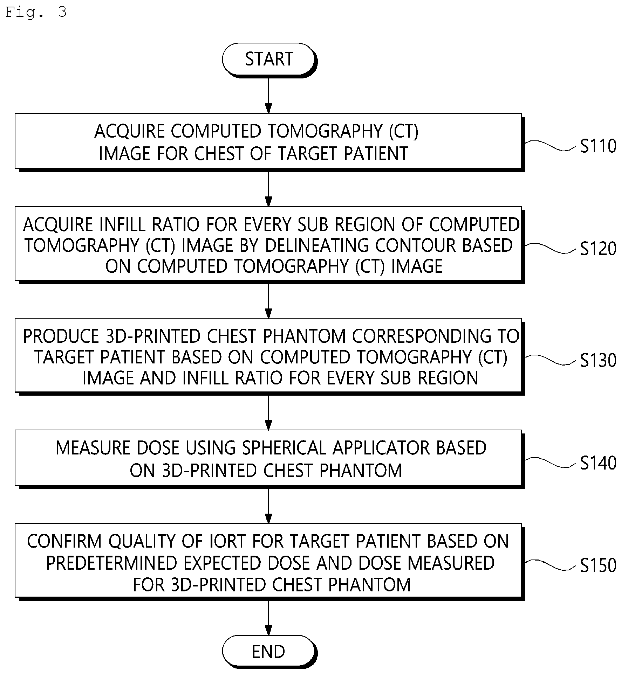

Now, a 3D-printed chest phantom manufacturing method for IORT according to an exemplary embodiment of the present disclosure will be described with reference to FIGS. 3 to 10.

Process of manufacturing 3D-printed chest phantom

FIG. 3 is a flowchart for explaining a 3D-printed chest phantom manufacturing method for IORT according to an exemplary embodiment of the present disclosure.

Referring to FIG. 3, the processor 110 of the chest phantom manufacturing apparatus 100 may acquire a computed tomography (CT) image for a chest of a target patient in step S110.

At this time, the chest phantom manufacturing apparatus 100 acquires a computed tomography (CT) image in the format of a digital imaging and communications in medicine (DICOM) standard.

Next, the processor 110 delineates a contour based on the computed tomography (CT) image to acquire an infill ratio for every sub region of the computed tomography (CT) image in step S120.

Here, the sub region may be one of a lung region and a soft tissue region.

That is, the processor 110 may acquire the infill ratio for every sub region of the computed tomography (CT) image using a predetermined conversion table.

Here, the conversion table represents a correlation between the infill ratio and a Hounsfield unit (HU) value. A water-equivalent phantom is used to verify manufacturer specification data of IORT (for example, INTRABEAM system) and verify the conversion table using the result.

Next, the processor 110 may manufacture the 3D-printed chest phantom corresponding to the target patient based on the computed tomography (CT) image and the infill ratio for every sub region using the 3D printing device 200 in step S130.

Next, the user may measure a dose of the target patient based on the manufactured 3D-printed chest phantom using a spherical applicator in step S140.

Here, as the spherical applicator, 35-mm spherical applicator may be used.

Thereafter, the user may confirm the quality of the IORT for the target patient based on a predetermined estimated dose and a dose measured for the 3D-printed chest phantom

Process of acquiring infill ratio for every sub region

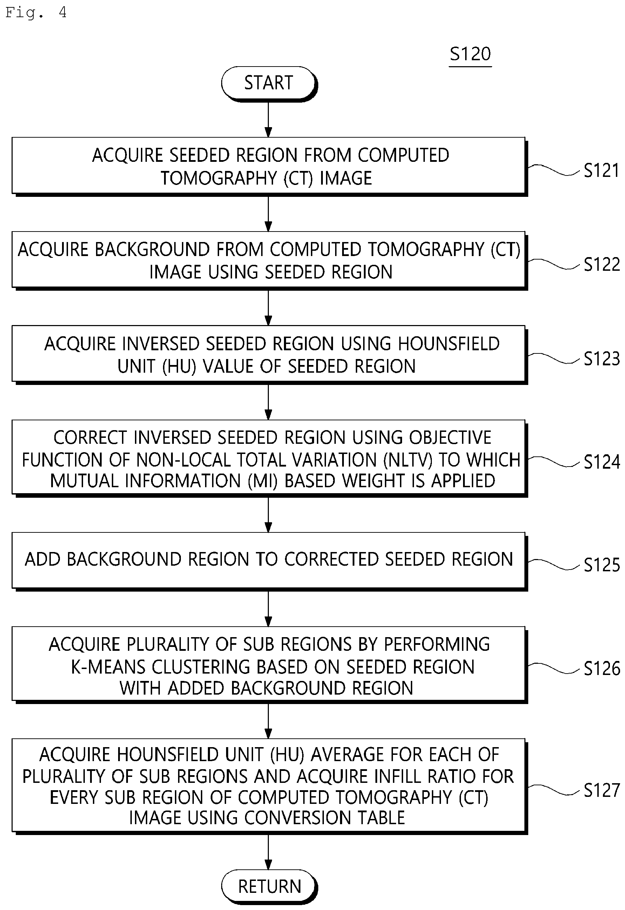

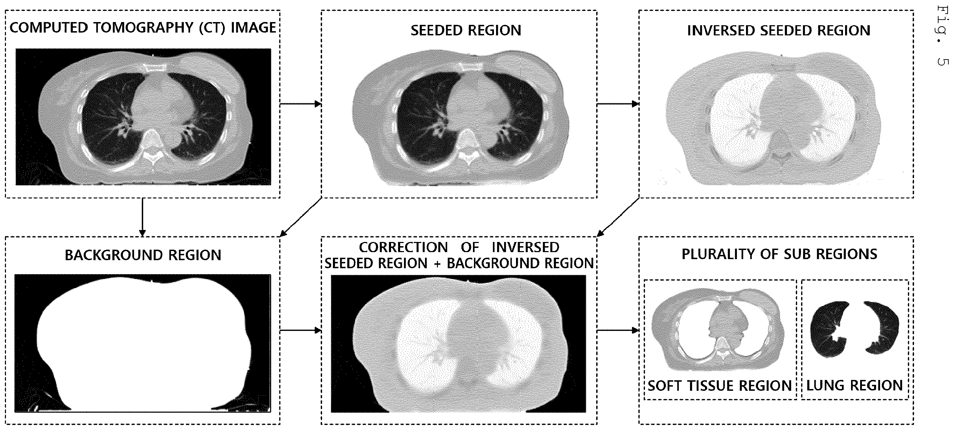

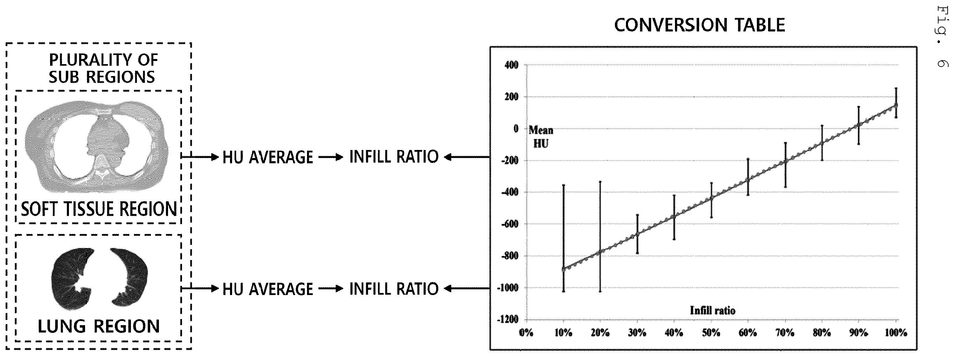

FIG. 4 is a flowchart for explaining a sub step of a step of acquiring an infill ratio illustrated in FIG. 3, FIG. 5 is a view for explaining an example of a process of acquiring a plurality of sub regions from a computed tomographic (CT) image according to an exemplary embodiment of the present disclosure, and FIG. 6 is a view for explaining an example of a process of acquiring an infill ratio for each of a plurality of sub regions illustrated in FIG. 5;

Referring to FIG. 4, the processor 110 may acquire a seeded region from the computed tomography (CT) image in step S121.

Here, the seeded region indicates a region corresponding to a chest of the target patient in the computed tomography (CT) image. For example, as illustrated in FIG. 5, the processor 110 may acquire a seeded region corresponding to a chest of the target patient from the computed tomography (CT) image.

Next, the processor 110 acquires a background region from the computed tomography (CT) image using the seeded region in step S122.

Here, the background region indicates a region which does not correspond to a chest of the target patient in the computed tomography (CT) image. For example, as illustrated in FIG. 5, the processor 110 may acquire the background region by removing the seeded region from the computed tomography (CT) image. That is, the computed tomography (CT) image may be configured by the seeded region corresponding to the chest of the target patient and the background region other than the seeded region.

Next, the processor 110 acquires an inversed seeded region using the Hounsfield unit (HU) value of the seeded region in step S123.

For example, as illustrated in FIG. 5, the processor 110 may acquire the inversed seeded region by replacing the Hounsfield unit (HU) value of each pixel of the seeded region with an inversed value.

Next, the processor 110 corrects the inversed seeded region using a non-local total variation (NLTV) objective function to which a mutual information (MI) based weight is applied in step S124.

Here, the objective function uses a weight between a voxel of the inversed seeded region and a voxel of a non-local patch included in a search set for a stationary patch. The weight may include a statistical scale value for one voxel acquired by means of the entropy and a factor which is spatially encoded for one voxel. The process of denoising an inversed seeded region using the non-local total variation (NLTV) to which the mutual information (MI) based weight is applied, according to the present disclosure, will be described in more detail below.

Next, the processor 110 may add the background region to the corrected seeded region in step S125.

For example, as illustrated in FIG. 5, the processor 110 may add the background region to the corrected seeded region using the non-local total variation (NLTV) to which the mutual information (MI) based weight is applied.

Next, the processor 110 may perform the K-means clustering based on the seeded region with the added background region to acquire a plurality of sub regions in step S126.

Here, the K-means clustering refers to an algorithm which groups data into K clusters. The K-means clustering is performed by processes of setting a number K of clusters -> setting an initial centroid -> assigning data to a cluster -> resetting (updating) a centroid -> reassigning data to a cluster. The "resetting (updating) of a centroid" and "reassigning data to a cluster" are repeated until the location of the centroid is not changed anymore. The K-means clustering is a known algorithm in the related art so that a detailed description will be omitted.

For example, as illustrated in FIG. 5, the processor 110 performs the K-means clustering (K = 3) on the seeded region with the added background region to acquire a plurality of sub regions (a soft tissue region, a lung region, and a background region).

Next, the processor 110 may acquire a Hounsfield unit (HU) average for each of the plurality of sub regions and acquire the infill ratio for every sub region of the computed tomography (CT) image using the conversion table in step S127.

For example, as illustrated in FIG. 6, the processor 110 acquires a Hounsfield unit (HU) average for the soft tissue region by averaging Hounsfield unit (HU) values of pixels which configure the soft tissue region and acquires a Hounsfield unit (HU) average for a lung region by averaging Hounsfield unit (HU) values of pixels which configure the lung region. The processor 110 acquires an infill ratio corresponding to the Hounsfield unit (HU) average for the soft tissue region using a predetermined conversion table and acquires an infill ratio corresponding to the Hounsfield unit (HU) average for the lung region using a predetermined conversion table.

At this time, there may be a conversion table for every type of IORT (for example, INTRABEAM system). In this case, the processor 100 may acquire an infill ratio for every sub region using the corresponding conversion table by means of identification information of the IORT (for example, INTRABEAM system).


Process of producing 3D-printed chest phantom

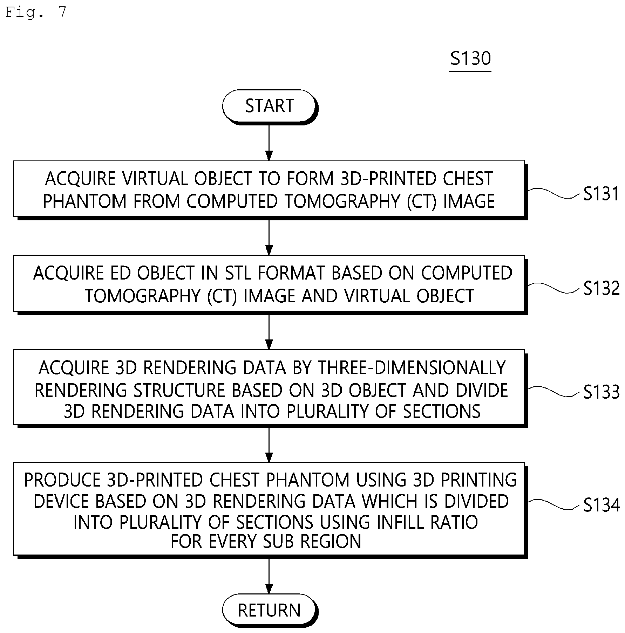

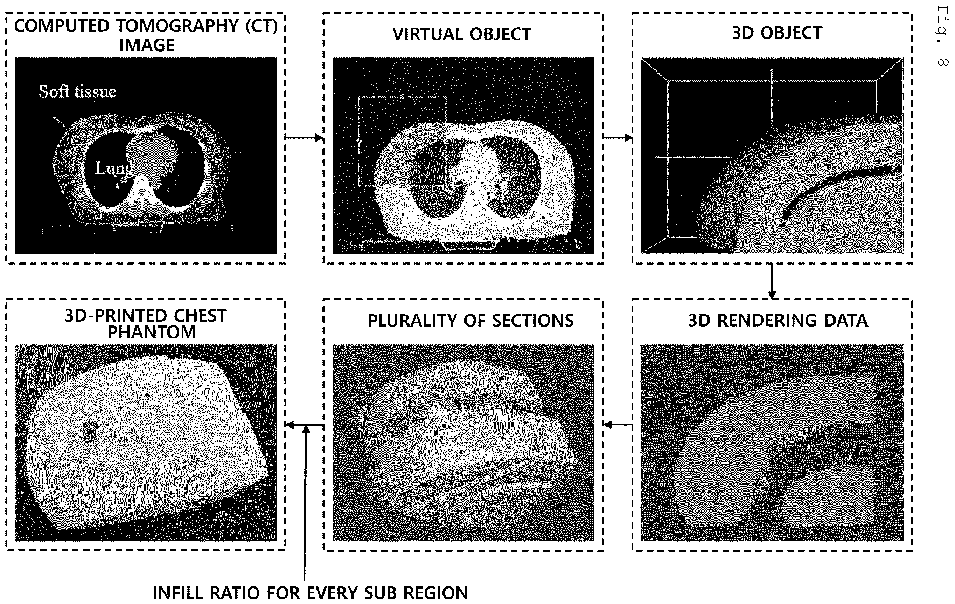

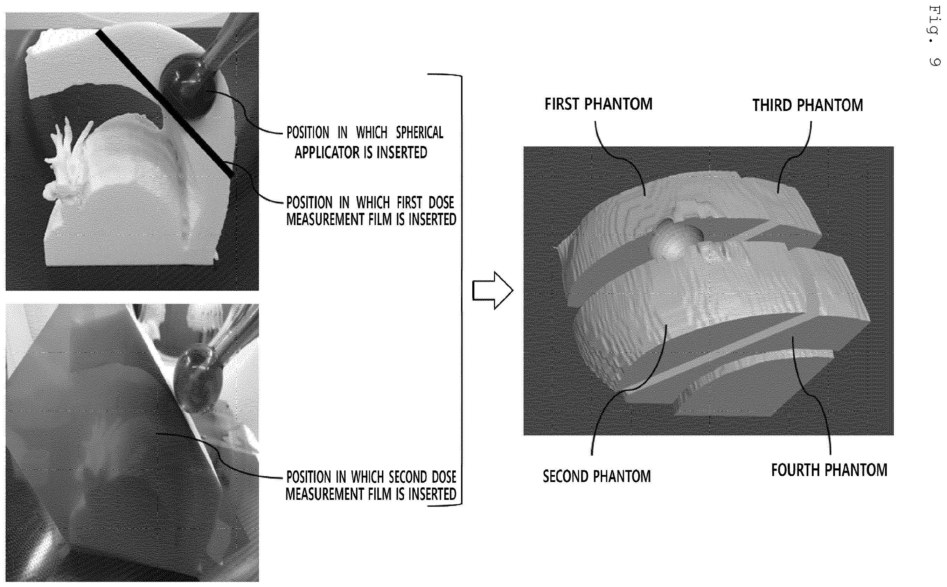

FIG. 7 is a flowchart for explaining a sub step of a step of manufacturing a 3D-printed chest phantom illustrated in FIG. 3, FIG. 8 is a view for explaining an example of a process of manufacturing a 3D-printed chest phantom according to an exemplary embodiment of the present disclosure, and FIG. 9 is a view for explaining an example of a process of acquiring a plurality of sections illustrated in FIG. 8.

Referring to FIG. 7, the processor 110 acquires a virtual object to form a 3D-printed chest phantom, from the computed tomography (CT) image in step S131.

Here, the virtual object refers to information which distinguishes a part to be simulated (for example, a part having a breast cancer) in the chest of the target patient. For example, as illustrated in FIG. 8, the processor 110 may acquire a virtual object which is a part to produce the 3D-printed chest phantom (a rectangular part in FIG. 8) from the computer tomography (CT) image. At this time, the virtual object may be designated from the computer tomography (CT) image displayed on a screen by the manipulation of an expert (for example, a doctor).

Next, the processor 110 may acquire a stereo lithography 3D object based on the computer tomography (CT) image and the virtual object in step S132.

Here, the STL format is one of standard formats of the 3D-modeled data.

For example, as illustrated in FIG. 8, the processor 110 may convert a virtual object (a part from which a chest phantom is produced) designated in the computer tomography (CT) image into a 3D object in an STL format.

Next, the processor 110 acquires 3D rendering data by three-dimensionally rendering a structure based on a 3D object and divides the 3D rendering data into a plurality of sections in step S133.

Here, the section refers to a unit produced by the 3D printing device 200.

For example, as illustrated in FIG. 8, the processor 110 acquires the 3D rendering data by rendering the 3D object in the STL format and divides the 3D rendering data into a plurality of sections.

At this time, the processor 110 may divide the 3D rendering data into a plurality of sections based on a position in which a spherical applicator is inserted, a position in which a first dose measurement film to check a dose distribution on a surface is inserted, and a position in which a second dose measurement film to check a dose distribution on a depth is inserted. The first dose measurement film and the second dose measurement film may be EBT3 films.

Here, a position in which the first dose measurement film is inserted may be determined so as to locate the first dose measurement film on a horizontal plane below a spherical shape into which the spherical applicator is inserted. A position in which the second dose measurement film is inserted may be determined to locate the second dose measurement film on a vertical plane below the horizontal plane (a plane on which the first dose measurement film is located).

For example, as illustrated in FIG. 9, the position in which the first dose measurement film is inserted may be determined so as to insert the first dose measurement film on a horizontal plane immediately below a spherical shape into which the spherical applicator is inserted and the position in which the second dose measurement film is inserted may be determined so as to insert the second dose measurement film on a vertical plane immediately below the horizontal plane (a plane into which the first dose measurement film is inserted). That is, the first dose measurement film and the second dose measurement film may be inserted below the spherical shape into which the spherical applicator is inserted to form a T shape. As described above, the first dose measurement film and the second dose measurement film are disposed on two planes (a vertical plane and a parallel plane) with respect to a beam axis to measure a surface dose and a depth dose. In other words, the 3D rendering data may be divided into a plurality of sections (for example, four sections) based on a region into which the spherical applicator is inserted, a region into which the first dose measurement film is inserted, and a region into which the second dose measurement film is inserted.

Next, the processor 110 may produce the 3D-printed chest phantom using the 3D printing device 200, based on the 3D rendering data divided into a plurality of sections, using an infill ratio for every sub region in step S134.

At this time, when the 3D-printed chest phantom is produced based on the 3D rendering data which is divided into the plurality of sections, the processor 110 may provide data to the 3D printing device 200 to distinguish a part corresponding to each sub region (a soft tissue region or a lung region) from each of the plurality of sections. Further, the processor 110 may also provide the infill ratio for every sub region (a soft tissue region or a lung region) to the 3D printing device 200. Accordingly, when a phantom for every section is produced based on the 3D rendering device, the 3D printing device 200 may produce the phantom by varying the infill ratio for every sub region (a soft tissue region or a lung region).

For example, as illustrated in FIG. 9, when the 3D rendering data is divided into four sections, the 3D printing device 100 produces a phantom (first sub phantom to fourth sub phantom of FIG. 9) for every section and assembles the phantom (first sub phantom to fourth sub phantom of FIG. 9) produced for every section to acquire a 3D-printed chest phantom corresponding to a target patient. That is, after inserting the spherical applicator, the first dose measurement film, and the second dose measurement film into the first sub phantom to fourth sub phantom, the first phantom to fourth phantom are assembled to produce the 3D-printed chest phantom corresponding to the target patient.

Process of denoising using mutual information based non-local total variation

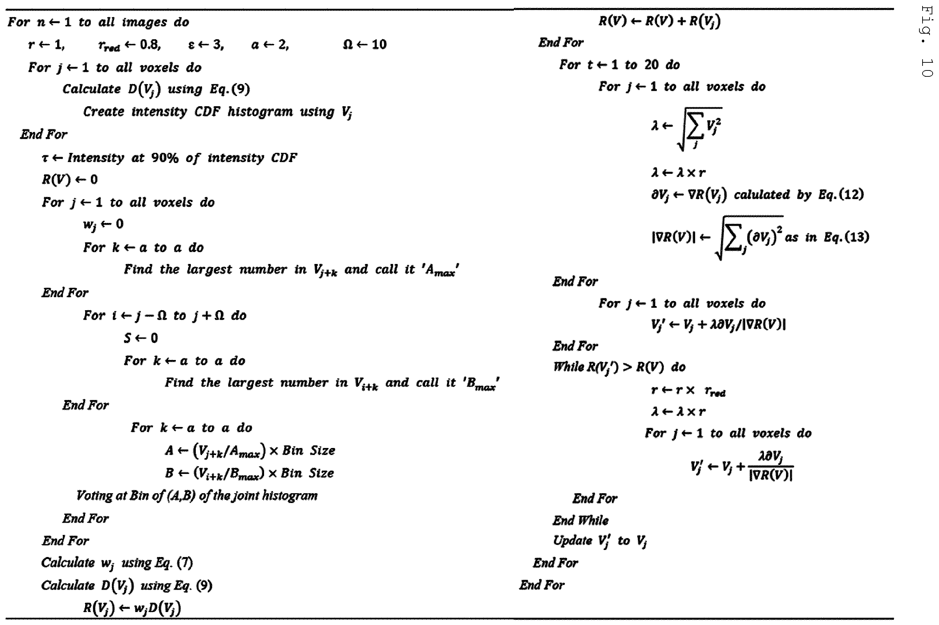

FIG. 10 is a view illustrating an example of a pseudo-code of mutual information based non-local total variation denoising according to an exemplary embodiment of the present disclosure.

The processor 110 according to the present disclosure improves an image of the image using a mutual information based non-local total variation (MI-NLTV) denoising technique. Here, the image refers to a seeded region.

Here, the mutual information MI refers to an amount of information indicating mutual dependence between two random variables. For example, when two random variables are completely independent (occurrence of an event A has no effect on the probability of the occurrence of an event B, and vice versa), the value is zero. When two random variables are closely related (when event A occurs, the probability of occurring B increases), the value is increased and when two random variables are inversely related (when event A occurs, the probability of occurring B decreases), the value becomes smaller. The local region processing is a method of updating a current target voxel only with a relationship between a target voxel to be currently corrected and adjacent voxels. In contrast, the non-local region processing is a method of designating not only adjacent voxels of the target voxel to be currently corrected, but also all voxels in the image to update the current target voxel by calculating a weight depending how similar voxel included in the group is to the target voxel. At this time, the similarity is calculated by comparing a small patch region including the target voxel and a small patch region for every voxel included in the group. When the range of the group voxel is set to an entire image size, it takes a long time to calculate so that it is generally implemented to set a search area to consider only the voxels included therein.

That is, the processor 110 corrects the inversed seeded region using a non-local total variation (NLTV) objective function to which a mutual information (MI) based weight is applied. Here, the image refers to a seeded region.

Here, the objective function of the mutual information based non-local total variation (MI-NLTV) uses a weight between a voxel of the image and a voxel of a non-local patch included in a search set for a stationary patch.

That is, the weight may include a statistical scale value for one voxel acquired by means of the entropy and a factor which is spatially encoded for one voxel.

Here, the statistical scale value may be acquired based on a first entropy indicating a marginal entropy for the stationary patch, a second entropy indicating a marginal entropy for a non-local patch, and a third entropy indicating a marginal entropy for the stationary patch and the non-local patch. That is, the first entropy is acquired based on a marginal probability distribution of the stationary patch, the second entropy is acquired based on a marginal probability distribution of the non-local patch, and the third entropy is acquired based on a joint probability distribution of the stationary patch and the non-local patch. At this time, the probability distribution used to acquire the first entropy, the second entropy, and the third entropy may be acquired using a joint histogram used to consider the relationship between intensities of the pixels in the stationary patch and the non-local patch. The joint histogram may be acquired by scaling intensity values of the patches according to a binning size in which a maximum value of each axis of the joint histogram is equal to a predetermined value.

The spatially encoded factor may include a normalization factor and a predetermined scaling factor. At this time, the normalization factor may be set based on a cumulative distribution function histogram acquired by accumulating intensities in each voxel of the image.

The process of denoising using the mutual information based non-local total variation (MI-NLTV) according to the present disclosure will be described in more detail below.

The present disclosure proposes a new type of non-local total variation (NLTV) based on a combination of mutual information (MI), that is, mutual information based non-local total variation (MI-NLTV). The present disclosure is based on statistical scale for similarity calculation between bins of the stationary patch and the non-local patch. The weight may be determined in terms of a statistical measure including a mutual information (MI) value between the entropies of the stationary patch and the non-local patch. The objective function of the MI-NLTV may be minimized based on the steepest gradient descent optimization to increase the difference between an actual structure and a noise. The minimization process may clean up a noise pixel without losing too much of a fine structure and details remaining in the image.

That is, in order to improve a signal difference between an outstanding feature and a unwanted noise by determining a similarity between the non-local patches, the total variation denoising process to which the weight is applied is applied to the image. When $\Phi_{i,j}$ is a region which is affected by a centroid of each patch, a state of the stationary patch is determined by calculating a statistical scale with the non-local patch included in the search set. MI is used as metric of image matching which does not require a linear relationship between the intensities. When the stationary patch and the non-local patch do not match, the MI has a low value. The stationary patch having a pattern including a noise-damaged pixel without having an outstanding feature is considered as a high entropy region. Therefore, it is likely to be in a smoothing state. In contrast, a region to be in a conservation state may be a patch having a low entropy and a high MI value. Further, the MI value may be equal to or smaller than an entropy of a marginal entropy of each patch. In consideration of this feature, the statistical scale $M(\Phi_{i,j})$ is defined as represented in the following Equation 1.

[Equation 1]

$$M(\Phi_{i,j})=\frac{MI(I_A,I_B^{\Omega})}{H(I_A)}$$

Here, $I_A$ indicates an area in the stationary patch and a search set for a non-local patch $I_B$ is defined as Q. $I_B^{\Omega}$ refers to all non-local patches included in a search set corresponding to the stationary patch.

$MI(I_A, I_B^{\Omega})$ is defined by the following Equation 2.

[Equation 2]

$$MI(I_{A,I_B})=H(I_A)+H(I_B^{\Omega})-H(I_A,I_B^{\Omega})$$

Here, $H(I_A)$ and $H(I_B^{\Omega})$ indicate marginal entropies of the stationary patch and the non-local patch. The entropies are defined as represented in Equation 3 to Equation 5.

[Equation 3]

$$H(I_A)=-\sum_{i\in I_A}p_i\log_2 p_i$$

[Equation 4]

$$H(I_B^\Omega)=-\sum_{i\in I_B^\Omega} p_i \log_2 p_i$$

[Equation 5]

$$H(I_A,I_B^\Omega)=-\sum_{i\in I_A}\sum_{j\in I_B^\Omega} p_{i,j} \log_2 p_{i,j}$$

Here, $p_i$ and $p_{i,j}$ are marginal probability distribution and joint probability distribution of $I_A$ and $I_B^\Omega$, respectively. In the case of the probability distribution required to calculate the entropies, the joint histogram is used to consider the relationship between intensities of the corresponding pixel in two stationary patches and the non-local patch. The joint histogram is acquired by scaling an intensity value of each patch according to the binning size in which a maximum value of each axis of the joint histogram is adjusted to be equal to a specific value.

A penalty having a different weight based on $M(\Phi_{i,j})$ is represented by the following Equation 6.

[Equation 6]

$$w_j=\exp(-(V_j/\tau)^\rho M(\Phi_{i,j}))$$

Here, an index i indicates an index of the non-local patch. An index j indicates an index of a voxel in the image and $V_j$ indicates a j-th voxel. $w_j$ indicates a weight between a current voxel j and a voxel in $\Omega$. A patch size is defined by $((2a+1)\times(2a+0))$ having a unit variance and a is set to 2 so that a patch size according to the present disclosure may be 5x5. The non-local search area may be 21 x 21 with a unit variance. $(V_j/\tau)^\rho$ is a factor which is spatially encoded for two hyper-parameters $\tau$ and j-th voxels having $\rho$. A function is to reduce a weighted averaging effect in a high intensity region to maintain a contrast. The local filtering parameter $\tau$ is a normalization factor for assuring that a ratio of $V_j/\tau$ exceeds 1. This may be set to 90% of a cumulative distribution function histogram by accumulating an intensity in each voxel of an image. The parameter $\rho$ (in an exemplary embodiment of the present disclosure, $\rho = 10$) is a scaling factor to assure a smaller weight for a higher intensity.

In the following Equation 7, minimization of the TV objective function to which the weight is applied indicates that edges having a higher contrast than that of the non-local patch are preserved and noise voxels having a lower contrast are smoothed.

[Equation 7]

$$R(V)=\sum_j R(V_j)=\sum_j w_j D(V_j)$$

Here, $D(V_j)$ is represented by Equation 9.

[Equation 8]

$$D(V_j)=\sqrt{(V_{(x,y)}-V_{(x-1,y)})^2+(V_{(x,y)}-V_{(x,y-1)})^2}$$

Here, $V_{(x,y)}$ is a voxel in a two-dimensional coordinate (x,y). The TV objective function to which a weight is applied is minimized based on an adaptive steepest gradient descent method having an adaptive step size represented by Equations 9 and 10.

[Equation 9]

$$V_j^{t+1}=V_j^t-\lambda\nabla R(V_j)/|\nabla R(V)|$$

[Equation 10]

$$\lambda = \gamma \sqrt{\sum_j \left( V_j^t \right)^2}$$

Here, $\lambda$ is an adaptive parameter which adjusts a step size and a smoothing degree is reduced as an iteration step is performed. In each step, a square root of all updated voxels is used so that $\lambda$ is applied as a smaller value as the number of iterations increases. In order to avoid local minimization due to sudden change, a scaling parameter $\gamma$ is used and an initial value is set to be 1.0.

When R(V) of the current iterative step is larger than R(V) of a previous step, this value is linearly reduced by multiplying a constant value ($r_{red}$ = 0.8). VR(V) is a gradient of an objective function R(V) in an j-th indexed pixel. A root-sum-square (RSS) | $\nabla$R(V) | of a gradient calculated in all the pixels is required to calculate a normalized gradient. To be sure, it may be represented by the following Equations 11 and 12.

[Equation 11]

$$\nabla R \left( V_j \right) = \frac{\partial R(V)}{\partial V_j} = \frac{\partial R(V)}{\partial V_{(x,y)}} = \left( \begin{array}{c} W_{(x,y)} \dfrac{2V_{(x,y)} - V_{(u-1,v)} - V_{(u,v-1)}}{\sqrt{\left(V_{(x,y)} - V_{(x-1,y)}\right)^2 + \left(V_{(x,y)} - V_{(x,y-1)}\right)^2}} \\ + W_{(x+1,y)} \dfrac{V_{(x,y)} - V_{(x+1,y)}}{\sqrt{\left(V_{(x+1,y)} - V_{(x,y)}\right)^2 + \left(V_{(x+1,y)} - V_{(x+1,y-1)}\right)^2}} \\ + W_{(x,y+1)} \dfrac{V_{(x,y)} - V_{(x,y+1)}}{\sqrt{\left(V_{(x,y+1)} - V_{(x-1,y+1)}\right)^2 + \left(V_{(x,y+1)} - V_{(x,y)}\right)^2}} \end{array} \right)$$

[Equation 12]

$$| \nabla R(V) | = \sqrt{\sum_j \left( \nabla R \left( V_j \right) \right)^2}$$

An optimal number of iterations for the adaptive steepest gradient descent step is finely adjusted to minimize a noise pixel while preserving an outstanding feature. In the exemplary embodiment of the present disclosure, a number of iteration of a step was set to 20. When all the above-mentioned items are considered, the pseudo-code of the MI-NLTV denoising is as illustrated in FIG. 10.

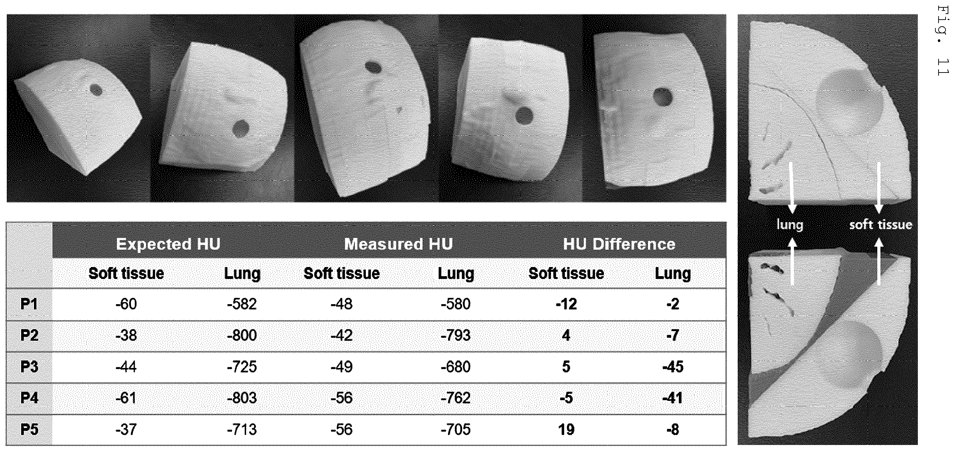

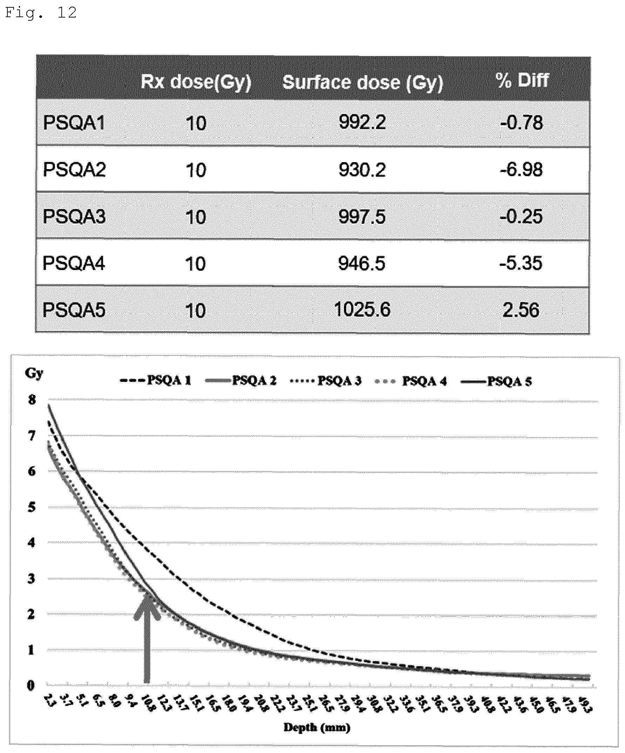

Now, a performance of a 3D-printed chest phantom manufacturing method for IORT according to an exemplary embodiment of the present disclosure will be described with reference to FIGS. 11 and 12.

FIG. 11 is a view for explaining a performance of a 3D-printed chest phantom manufacturing method for IORT according to an exemplary embodiment of the present disclosure which illustrates a result of comparing a dose measured using a patient-specific 3D-printed chest phantom and a predicted dose and FIG. 12 is a view for explaining a performance of a 3D-printed chest phantom manufacturing method for IORT according to an exemplary embodiment of the present disclosure which illustrates a surface dose and a depth dose which are measured using a patient-specific 3D-printed chest phantom and a predicted dose.

A 3D-printed chest phantom for each of five patients P1 to P5 is produced based on a computer tomography (CT) image for a chest of five patients P1 to P5 and a patient-specific quality assurance (PSQA) for five patients P1 to P5 are performed using the 3D chest phantom for each of five patients P1 to P5.

Referring to FIG. 11, it is confirmed that a dose change in a treatment planning(that is, a difference between an expected dose and a measured dose) is within $\pm$1% in consideration of an allowable error of $\pm$20 HU for a soft tissue and an allowable error of $\pm$50 HU for a lung.

Referring to FIG. 12, it is confirmed that a dose irradiated on a surface of the spherical applicator has a percent error of -2.16% $\pm$ 3.91% between the measured dose and an expected dose (prescription dose). It is further confirmed that the depth dose is reduced to 75% at a depth of 10 mm from a surface of the spherical applicator.

As described above, according to the present disclosure, a 3D-printed chest phantom for every patient is manufactured and the IORT related dose distribution may be successfully simulated in advance using the manufactured 3D-printed chest phantom for every patient. By doing this, it is possible to not only predict a depth dose in various distances from the spherical applicator, but also verify a dose by comparing the expected dose and the measured dose.

Accordingly, the present disclosure may provide a new opportunity for IORT development.

The operation according to the exemplary embodiment of the present disclosure may be implemented as a program instruction which may be executed by various computers to be recorded in a computer readable storage medium. The computer readable storage medium indicates an arbitrary medium which participates to provide a command to a processor for execution. The computer readable storage medium may include solely a program command, a data file, and a data structure or a combination thereof. For example, the computer readable medium may include a magnetic medium, an optical recording medium, and a memory. The computer program may be distributed on a networked computer system so that the computer readable code may be stored and executed in a distributed manner. Functional programs, codes, and code segments for implementing the present embodiment may be easily inferred by programmers in the art to which this embodiment belongs.

The present embodiments are provided to explain the technical spirit of the present embodiment and the scope of the technical spirit of the present embodiment is not limited by these embodiments. The protection scope of the present embodiments should be interpreted based on the following appended claims and it should be appreciated that all technical spirits included within a range equivalent thereto are included in the protection scope of the present embodiments.

**Claims**

1. A 3D-printed chest phantom manufacturing method for IORT, comprising:

   acquiring a computed tomography (CT) image for a chest of a target patient;
   acquiring an infill ratio for every sub region of the computed tomography (CT) image by delineating a contour based on the computed tomography (CT) image;
   producing a 3D-printed chest phantom corresponding to the target patient based on the computed tomography (CT) image and the infill ratio for every sub region.

2. The 3D-printed chest phantom manufacturing method according to claim 1, wherein the producing of a 3D-printed chest phantom includes:

   acquiring a virtual object to form the 3D-printed chest phantom from the computed tomography (CT) image;
   acquiring a 3D object in a stereo lithography (STL) format based on the computed tomography (CT) image and the virtual object;
   acquiring 3D rendering data by three-dimensionally rendering a structure based on the 3D object and dividing the 3D rendering data into a plurality of sections; and
   producing the 3D-printed chest phantom using a 3D printing device, based on the 3D rendering data which is divided into the plurality of sections using the infill ratio for every sub region.

3. The 3D-printed chest phantom manufacturing method according to claim 2, wherein in the dividing into the plurality of sections, the 3D rendering data is divided into the plurality of sections based on a position in which a spherical applicator is inserted, a position in which a first dose measurement film to check a dose distribution on a surface is inserted, and a position in which a second dose measurement film to check a dose distribution on a depth is inserted.

4. The 3D-printed chest phantom manufacturing method according to claim 3, wherein a position in which the first dose measurement film is inserted is determined so as to locate the first dose measurement film on a lower horizontal plane of a spherical shape into which the spherical applicator is inserted and a position in which the second dose measurement film is inserted is determined to locate the second dose measurement film on a vertical plane below the horizontal plane.

5. The 3D-printed chest phantom manufacturing method according to claim 1, wherein in the acquiring of an infill ratio, the infill ratio is acquired for every sub region of the computed tomography (CT) image using a predetermined conversion table representing a correlation between the infill ratio and a Hounsfield unit (HU) value.

6. The 3D-printed chest phantom manufacturing method according to claim 5, wherein the acquiring of an infill ratio includes:

   acquiring a seeded region from the computed tomography (CT) image;
   acquiring a background region from the computed tomography (CT) image using the seeded region;

acquiring an inversed seeded region using the Hounsfield unit (HU) value of the seeded region;

correcting the inversed seeded region using a non-local total variation (NLTV) objective function to which a mutual information (MI) based weight is applied;

adding the background region to the corrected seeded region;

acquiring a plurality of sub regions by performing the K-means clustering based on the seeded region with the added background region; and

acquiring a Hounsfield unit (HU) average for each of the plurality of sub regions and acquiring an infill ratio for every sub region of the computed tomography (CT) image using the conversion table.

7. The 3D-printed chest phantom manufacturing method according to claim 6, wherein the objective function uses a weight between a voxel of the inversed seeded region and a voxel of a non-local patch included in a search set for a stationary patch and the weight includes a statistical scale value for one voxel acquired by means of the entropy and a factor which is spatially encoded for one voxel.

8. The 3D-printed chest phantom manufacturing method according to claim 1, wherein the sub region is one of a lung region and a soft tissue region.

9. A computer program stored in a computer readable storage medium to allow a computer to execute the 3D-printed chest phantom manufacturing method for IORT according to any one of claims 1 to 8.

10. A chest phantom manufacturing apparatus which manufactures a 3D-printed chest phantom for intraoperative radiotherapy (IORT), comprising:

a memory which stores one or more programs to manufacture the 3D-printed chest phantom; and

one or more processors which perform an operation for manufacturing the 3D-printed chest phantom according to the one or more programs stored in the memory,

wherein the processor acquires a computed tomography (CT) image for a chest of a target patient, delineates a contour based on the computed tomography (CT) image to acquire an infill ratio for every sub region of the computed tomography (CT) image, and produces a 3D-printed chest phantom corresponding to the target patient based on the computed tomography (CT) image and the infill ratio for every sub region.

11. The 3D-printed chest phantom manufacturing apparatus according to claim 10, wherein the processor acquires a virtual object to form the 3D-printed chest phantom from the computed tomography (CT) image, acquires a 3D object in a stereo lithography (STL) format based on the computed tomography (CT) image and the virtual object, acquires 3D rendering data by three-dimensionally rendering a structure based on the 3D object and divides the 3D rendering data into a plurality of sections, and produces the 3D-printed chest phantom using a 3D printing device, based on the 3D rendering data which is divided into the plurality of sections using the infill ratio for every sub region.

12. The 3D-printed chest phantom manufacturing apparatus according to claim 11, wherein the processor divides the 3D rendering data into the plurality of sections based on a position in which a spherical applicator is inserted, a position in which a first dose measurement film to check a dose distribution on a surface is inserted, and a position in which a second dose measurement film to check a dose distribution on a depth is inserted.

13. The 3D-printed chest phantom manufacturing apparatus according to claim 10, wherein the processor acquires the infill ratio for every sub region of the computed tomography (CT) image using a predetermined conversion table representing a correlation between the infill ratio and a Hounsfield unit (HU) value.

Fig. 1

PATIENT-SPECIFIC QUALITY
ASSURANCE DEVICE
(100)

3D PRINTING DEVICE
(200)

Fig. 2

100

COMPUTER READABLE
STORAGE MEDIUM
(130)

PROGRAM
(131)

150

PROCESSOR
(110)

INPUT/OUTPUT
INTERFACE (170)

COMMUNICATION
INTERFACE (190)

Fig. 3

```
                    ( START )
                        |
                        v
+------------------------------------------------+
|   ACQUIRE COMPUTED TOMOGRAPHY (CT)             |
|   IMAGE FOR CHEST OF TARGET PATIENT            |  ~ S110
+------------------------------------------------+
                        |
                        v
+------------------------------------------------+
| ACQUIRE INFILL RATIO FOR EVERY SUB REGION      |
| OF COMPUTED TOMOGRAPHY (CT) IMAGE BY           |
| DELINEATING CONTOUR BASED ON COMPUTED          |  ~ S120
| TOMOGRAPHY (CT) IMAGE                          |
+------------------------------------------------+
                        |
                        v
+------------------------------------------------+
| PRODUCE 3D-PRINTED CHEST PHANTOM               |
| CORRESPONDING TO TARGET PATIENT BASED ON       |
| COMPUTED TOMOGRAPHY (CT) IMAGE AND INFILL      |  ~ S130
| RATIO FOR EVERY SUB REGION                     |
+------------------------------------------------+
                        |
                        v
+------------------------------------------------+
| MEASURE DOSE USING SPHERICAL APPLICATOR        |
| BASED ON 3D-PRINTED CHEST PHANTOM              |  ~ S140
+------------------------------------------------+
                        |
                        v
+------------------------------------------------+
| CONFIRM QUALITY OF IORT FOR TARGET PATIENT     |
| BASED ON PREDETERMINED EXPECTED DOSE AND       |  ~ S150
| DOSE MEASURED FOR 3D-PRINTED CHEST PHANTOM     |
+------------------------------------------------+
                        |
                        v
                    ( END )
```

Fig. 4

S120

START

ACQUIRE SEEDED REGION FROM COMPUTED TOMOGRAPHY (CT) IMAGE — S121

ACQUIRE BACKGROUND FROM COMPUTED TOMOGRAPHY (CT) IMAGE USING SEEDED REGION — S122

ACQUIRE INVERSED SEEDED REGION USING HOUNSFIELD UNIT (HU) VALUE OF SEEDED REGION — S123

CORRECT INVERSED SEEDED REGION USING OBJECTIVE FUNCTION OF NON-LOCAL TOTAL VARIATION (NLTV) TO WHICH MUTUAL INFORMATION (MI) BASED WEIGHT IS APPLIED — S124

ADD BACKGROUND REGION TO CORRECTED SEEDED REGION — S125

ACQUIRE PLURALITY OF SUB REGIONS BY PERFORMING K-MEANS CLUSTERING BASED ON SEEDED REGION WITH ADDED BACKGROUND REGION — S126

ACQUIRE HOUNSFIELD UNIT (HU) AVERAGE FOR EACH OF PLURALITY OF SUB REGIONS AND ACQUIRE INFILL RATIO FOR EVERY SUB REGION OF COMPUTED TOMOGRAPHY (CT) IMAGE USING CONVERSION TABLE — S127

RETURN

Fig. 5

CONVERSION TABLE

PLURALITY OF SUB REGIONS

SOFT TISSUE REGION

HU AVERAGE → INFILL RATIO ←

LUNG REGION

HU AVERAGE → INFILL RATIO ←

Mean HU

Infill ratio

400
200
0
-200
-400
-600
-800
-1000
-1200

0%  10%  20%  30%  40%  50%  60%  70%  80%  90%  100%

Fig. 6

EP 4 005 632 A1

Fig. 7

S130

```
        ┌─────────────┐
        │    START    │
        └──────┬──────┘
               │
               ▼
┌──────────────────────────────────────────────┐
│   ACQUIRE VIRTUAL OBJECT TO FORM 3D-PRINTED   │      ╮─ S131
│    CHEST PHANTOM FROM COMPUTED TOMOGRAPHY      │
│              (CT) IMAGE                        │
└──────────────────────┬───────────────────────┘
                       │
                       ▼
┌──────────────────────────────────────────────┐
│   ACQUIRE ED OBJECT IN STL FORMAT BASED ON    │      ╮─ S132
│   COMPUTED TOMOGRAPHY (CT) IMAGE AND VIRTUAL  │
│                  OBJECT                        │
└──────────────────────┬───────────────────────┘
                       │
                       ▼
┌──────────────────────────────────────────────┐
│  ACQUIRE 3D RENDERING DATA BY THREE-          │      ╮─ S133
│  DIMENSIONALLY RENDERING STRUCTURE BASED ON   │
│  3D OBJECT AND DIVIDE 3D RENDERING DATA INTO  │
│          PLURALITY OF SECTIONS                │
└──────────────────────┬───────────────────────┘
                       │
                       ▼
┌──────────────────────────────────────────────┐
│  PRODUCE 3D-PRINTED CHEST PHANTOM USING 3D    │      ╮─ S134
│  PRINTING DEVICE BASED ON 3D RENDERING DATA   │
│  WHICH IS DIVIDED INTO PLURALITY OF SECTIONS  │
│  USING INFILL RATIO FOR EVERY SUB REGION      │
└──────────────────────┬───────────────────────┘
                       │
                       ▼
        ┌─────────────┐
        │   RETURN    │
        └─────────────┘
```

Fig. 8

Fig. 9

THIRD PHANTOM

FOURTH PHANTOM

FIRST PHANTOM

SECOND PHANTOM

POSITION IN WHICH SPHERICAL APPLICATOR IS INSERTED

POSITION IN WHICH FIRST DOSE MEASUREMENT FILM IS INSERTED

POSITION IN WHICH SECOND DOSE MEASUREMENT FILM IS INSERTED

Fig. 10

EP 4 005 632 A1

```
For n ← 1 to all images do
    r ← 1,     r_red ← 0.8,     ε ← 3,     a ← 2,          Ω ← 10
    For j ← 1 to all voxels do
        Calculate D(V_j) using Eq.(9)
            Create intensity CDF histogram using V_j
    End For
        τ ← Intensity at 90% of intensity CDF
        R(V) ← 0
        For j ← 1 to all voxels do
            w_j ← 0
            For k ← a to a do
                Find the largest number in V_{j+k} and call it 'A_max'
    End For
            For i ← j − Ω to j + Ω do
                S ← 0
                For k ← a to a do
                    Find the largest number in V_{i+k} and call it 'B_max'
        End For
                For k ← a to a do
                    A ← (V_{j+k}/A_max) × Bin Size
                    B ← (V_{i+k}/B_max) × Bin Size
                Voting at Bin of (A,B) of the joint histogram
            End For
        End For
    Calculate w_j using Eq. (7)
    Calculate D(V_j) using Eq. (9)
            R(V_j) ← w_j D(V_j)
```

```
            R(V) ← R(V) + R(V_j)
    End For
        For t ← 1 to 20 do
            For j ← 1 to all voxels do

                λ ← sqrt( Σ_j V_j^2 )

                λ ← λ × r
                ∂V_j ← ∇R(V_j) caluated by Eq.(12)

                |∇R(V)| ← sqrt( Σ_j (∂V_j)^2 )  as in Eq.(13)

        End For
            For j ← 1 to all voxels do
                V_j' ← V_j + λ∂V_j/|∇R(V)|
        End For
        While R(V_j') > R(V) do
                r ← r × r_red
                λ ← λ × r
                For j ← 1 to all voxels do

                    V_j' ← V_j + λ∂V_j/|∇R(V)|

            End For
        End While
        Update V_j' to V_j
    End For
End For
```

$$\lambda \leftarrow \sqrt{\sum_{j} V_j^2}$$

$$|\nabla R(V)| \leftarrow \sqrt{\sum_{j} (\partial V_j)^2}$$

$$V_j' \leftarrow V_j + \frac{\lambda \partial V_j}{|\nabla R(V)|}$$

Fig. 11

| | Expected HU | | Measured HU | | HU Difference | |
|---|---|---|---|---|---|---|
| | Soft tissue | Lung | Soft tissue | Lung | Soft tissue | Lung |
| P1 | -60 | -582 | -48 | -580 | -12 | -2 |
| P2 | -38 | -800 | -42 | -793 | 4 | -7 |
| P3 | -44 | -725 | -49 | -680 | 5 | -45 |
| P4 | -61 | -803 | -56 | -762 | -5 | -41 |
| P5 | -37 | -713 | -56 | -705 | 19 | -8 |

Fig. 12

| | Rx dose(Gy) | Surface dose (Gy) | % Diff |
|---|---|---|---|
| PSQA1 | 10 | 992.2 | -0.78 |
| PSQA2 | 10 | 930.2 | -6.98 |
| PSQA3 | 10 | 997.5 | -0.25 |
| PSQA4 | 10 | 946.5 | -5.35 |
| PSQA5 | 10 | 1025.6 | 2.56 |

Europäisches
Patentamt

European
Patent Office

Office européen
des brevets

# EUROPEAN SEARCH REPORT

Application Number

EP 21 21 0152

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KAIRN T ET AL: "Quasi-simultaneous 3D printing of muscle-, lung- and bone-equivalent media: a proof-of-concept study", PHYSICAL AND ENGINEERING SCIENCES IN MEDICINE, SPRINGER INTERNATIONAL PUBLISHING, CHAM, vol. 43, no. 2, 20 April 2020 (2020-04-20), pages 701-710, XP037163498, ISSN: 2662-4729, DOI: 10.1007/S13246-020-00864-5 [retrieved on 2020-04-20] * page 702 - page 704 * | 1-13 | INV. A61N5/10 B29C64/386 G09B23/30 G16H20/40 G16H50/50 |
| X | COLIEN HAZELAAR ET AL: "Using 3D printing techniques to create an anthropomorphic thorax phantom for medical imaging purposes", MEDICAL PHYSICS., vol. 45, no. 1, 1 January 2018 (2018-01-01), pages 92-100, XP055611127, US ISSN: 0094-2405, DOI: 10.1002/mp.12644 * the whole document * | 1-13 | |
| A | DANCEWICZ O L ET AL: "Radiological properties of 3D printed materials in kilovoltage and megavoltage photon beams", PHYSICA MEDICA, ACTA MEDICA EDIZIONI E CONGRESSI, ROME, IT, vol. 38, 26 May 2017 (2017-05-26), pages 111-118, XP085065311, ISSN: 1120-1797, DOI: 10.1016/J.EJMP.2017.05.051 * Sections 2 and 3 * | 1-13 | TECHNICAL FIELDS SEARCHED (IPC) A61N G09D B29C G09B G16H |

The present search report has been drawn up for all claims

| Place of search | Date of completion of the search | Examiner |
|---|---|---|
| Munich | 11 April 2022 | Rivera Farina, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)